# EUROPÄISCHE PATENTSCHRIFT

(11) **EP 0 889 361 B1**
(45) Veröffentlichungstag und Bekanntmachung des Hinweises auf die Patenterteilung: **23.01.2002**
(21) Anmeldenummer: 98111154.5
(22) Anmeldetag: 17.06.1998
(51) Int. Cl.: G03F 7/029, C07D 311/06

(54) **Initiatoren für die kationische Polymerisation**
Initiators for cationic polymerization
Initiateurs pour la polymerisation cationique

(30) Priorität: 30.06.1997 DE 19727820
(43) Veröffentlichungstag der Anmeldung: 07.01.1999
(73) Patentinhaber: SIEMENS AKTIENGESELLSCHAFT, 80333 München (DE)
(72) Erfinder: Schön, Lothar, Dipl.-Ing. (FH), 91077 Neunkirchen (DE); Rogler, Wolfgang, Dr., 91096 Möhrendorf (DE); Muhrer, Volker, Dipl.-Ing. (FH), 90768 Fürth (DE); Fedtke, Manfred, Prof. Dr., 06217 Merseburg (DE); Palinsky, Andreas, Dipl.-Ing., 30823 Garbsen (DE)

(56) Entgegenhaltungen:
- EP-A- 0 550 337
- EP-A- 0 636 939
- EP-A- 0 704 764
- DE-A- 2 704 368
- US-A- 5 141 969

## Beschreibung

Die Erfindung betrifft neue Initiatoren für die kationische Polymerisation sowie diese Initiatoren enthaltende kationisch härtbare Reaktionsharzmischungen und deren Verwendung.

Mittels UV-Licht nach einem kationischen Mechanismus härtbare Reaktionsharzmischungen erlangen eine zunehmende technische Bedeutung, weil sie zu Formstoffen mit ausgezeichneten thermisch-mechanischen Eigenschaften führen. Die chemische Basis dieser Reaktionsharzmischungen sind Verbindungen, welche Oxiranringe enthalten, wie Epoxidharze, und/oder Vinylether. Während sich Reaktionsharzmischungen auf der Basis von Vinylethern durch eine hohe Reaktionsgeschwindigkeit (bei der Härtung) auszeichnen, erweisen sich solche auf der Basis von Epoxiden wegen des günstigeren Schwundverhaltens bei der Härtung als vorteilhaft.

Bei der Härtung der Reaktionsharzmischungen wird das UV-Licht von einem Photoinitiator absorbiert, der aufgrund von Folgereaktionen Carbokationen bzw. Protonen bildet; dies sind die eigentlichen aktiven Spezies für den Start der Polymerisation. Übliche Photoinitiatoren für die kationische Polymerisation sind beispielsweise Triarylsulfoniumsalze. Diese weisen zwar bei UV-Bestrahlung eine gute Reaktivität auf, thermisch (d.h. bei einer Temperaturerhöhung) sind sie jedoch sehr stabil und deshalb nicht in der Lage, die kationische Polymerisation thermisch zu initiieren.

Eine thermische Härtung ist immer dann erforderlich, wenn nicht alle Harzbereiche belichtet werden können. Dies ist beispielsweise der Fall, wenn dickere Schichten erzeugt werden müssen oder in der Reaktionsharzmischung lichtstreuende oder -absorbierende Zusatzstoffe, wie Füllstoffe, Pigmente und Farbstoffe, enthalten sind. Dann wird nämlich das Licht in den oberflächennahen Schichtbereichen sehr stark absorbiert bzw. gestreut, so daß das in tiefere Schichtbereiche transmittierte Licht nicht ausreicht, um eine (vollständige) Härtung zu bewirken.

Ferner ist eine UV-Härtung nicht möglich, wenn verfahrensbedingt Bereiche vorhanden sind, die einer direkten Bestrahlung nicht zugänglich sind. Bei der Verklebung von nicht-transparenten Fügeteilen sowie von elektronischen Bauelementen und Baugruppen wird zunächst der Kleber appliziert und dann das Bauteil aufgesetzt. Durch Bestrahlung mit UV-Licht können dabei nur die Randbereiche gehärtet werden, an denen der Kleber hervorquillt, unter dem Bauteil muß die Härtung dagegen durch einen zusätzlichen Prozeß, beispielsweise thermisch induziert, erfolgen. Vergleichbar ist die Situation, wenn Bauelemente und Baugruppen zum Schutz vor Umgebungseinflüssen mit einem Schutzlack versehen werden. Aufgrund der Kapillarkräfte migriert der Lack nämlich unter die Bauteile und kann dort wiederum nicht durch Belichtung gehärtet werden.

UV-härtbare Reaktionsharzmischungen können zur Herstellung komplexer Kunststoffmodelle durch Stereolithographie mit Hilfe von 3D-CAD-Daten eingesetzt werden. Dabei wird die Oberfläche eines flüssigen, durch Laserlicht härtbaren Photopolymers mit einem rechnergesteuerten Laserstrahl bildmäßig belichtet, wobei eine erste Schicht des herzustellenden dreidimensionalen Gebildes aushärtet. Anschließend wird diese Schicht mit frischem Photopolymer beschichtet und erneut bildmäßig mit dem Laser belichtet. Hierbei entsteht eine zweite gehärtete Schicht des dreidimensionalen Gebildes, die sich mit der ersten verbindet. Dieser Prozeß wird so lange fortgesetzt, bis das gesamte Gebilde hergestellt ist, das dabei in das Photopolymerbad hineinwächst. Das auf diese Weise gebildete, nur teilweise ausgehärtete "Grünteil" wird anschließend durch längere Belichtung mit UVA-Licht weitgehend ausgehärtet.

Bei diesem Verfahren werden vorteilhaft Photopolymere auf der Basis von Epoxidharzen eingesetzt, die gegenüber solchen auf Acrylatbasis ein günstigeres Schwundverhalten zeigen, so daß eine höhere Maß- und Formhaltigkeit erreicht werden kann. Außerdem ist eine Nachhärtung der teilgehärteten Grünteile auch durch Temperaturerhöhung möglich. Diese thermische Härtung gelingt aber nur dann, wenn alle zu verfestigenden Bereiche vorher belichtet wurden. Eine rein thermische Härtung ist bei konventionellen Photopolymeren mit Triarylsulfoniumsalzen dagegen nicht möglich.

Die Möglichkeit einer thermischen Härtung wird aber deshalb angestrebt, weil sich bei einer partiellen Belichtung, beispielsweise zur Erzeugung von Konturen und Gitternetzen, eine erhebliche Zeiteinsparung - im Vergleich zu einer vollflächigen Belichtung - ergibt. Voraussetzung ist dabei allerdings, daß die nicht-belichteten Bereiche durch einen nachfolgenden thermischen Prozeß ausgehärtet werden können, da sonst nur ungenügende Formstoffeigenschaften erreicht werden. Eine Nachbelichtung dieser Bereiche im Inneren dicker Teile ist nicht möglich, weil das Licht durch den Photoinitiator in den Randschichten absorbiert wird und somit für einen photochemischen Prozeß im Inneren der Teile nicht zur Verfügung steht.

Die Härtungsgeschwindigkeit bei der kationischen Polymerisation von Epoxidharzen ist geringer als diejenige bei der Polymerisation von Acrylaten, und außerdem ist zur Härtung eine höhere UV-Dosis erforderlich. Deshalb müssen möglichst leistungsstarke UV-Laser eingesetzt werden. Diese stehen beispielsweise mit Argon-Ionen-Lasern (mit Wellenlängen von 351 und 364 nm) sowie mit frequenzverdreifachten Nd:YAG-Lasern (mit einer Wellenlänge von 351 nm) auch zur Verfügung, bei diesen Wellenlängen ist aber das Absorptionsvermögen der üblicherweise eingesetzten Triarylsulfoniumsalze zu gering, um effektiv Kationen bzw. Protonen bilden zu können.

Aus der EP 0 370 693 A2 sind Oniumsalze zur Verwendung als Photoinitiatoren bekannt, die bei Bestrahlung mit sichtbarem Licht eine Brönsted-Säure bilden. Dies sind Sulfonium-, Arsonium-, Ammonium- und Phosphoniumsalze (mit einem S-, As-, N- bzw. P-Atom), die einen Chromophor enthalten, welcher sichtbares Licht absorbiert, wobei der Chromophor vom S-, As-, N- bzw. P-Atom durch eine isolierende Gruppe getrennt ist, welche eine π-Resonanz (zwischen dem Chromophor und den anderen Substituenten) verhindert. Die Oniumsalze enthalten ferner wenigstens einen Substituenten, der eine elektronenziehende Gruppierung darstellt und ein unbesetztes Molekülorbital auf niedrigerem Energieniveau aufweist als der lichtabsorbierende Chromophor. Beispiele für derartige Oniumsalze sind: 4-Cyanobenzyl-2-[5-naphthacenyl]benzylphenylsulfoniumhexafluorophosphat und -trifluormethansulfonat sowie Phenyl-p-cyanobenzyl-4-[6,7-dimethoxycumarin-methyl]sulfonium-hexafluorophosphat und -trifluormethansulfonat. Eine Aktivierung der Oniumsalze durch UV-Licht erfolgt nicht, ebensowenig eine rein thermische Bildung der Brönsted-Säuren, d.h. ohne vorangegangene Belichtung.

Aufgabe der Erfindung ist es, Initiatoren für die kationische Polymerisation, d.h. für die Härtung kationisch polymerisierbarer Reaktionsharzmischungen, anzugeben, die sowohl durch UV-Bestrahlung, insbesondere im Bereich von etwa 300 bis 400 nm, als auch thermisch aktivierbar sind; außerdem sollen die Initiator enthaltenden Reaktionsharzmischungen lagerstabil sein.

Dies wird erfindungsgemäß dadurch erreicht, daß die Initiatoren Verbindungen folgender Struktur sind: 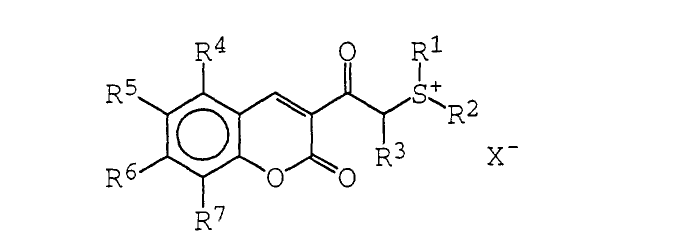 wobei folgendes gilt:
R¹ und R² sind - unabhängig voneinander - Alkyl mit 1 bis 9 C-Atomen (linear oder verzweigt) oder Cycloalkyl mit 4 bis 9 C-Atomen oder bilden zusammen eine divalente aliphatische Gruppierung mit 4 bis 7 C-Atomen, d.h. zusammen mit dem S-Atom einen heterocyclischen Ring,
R³ ist H oder Alkyl mit 1 bis 9 C-Atomen (linear oder verzweigt),
R⁴, R⁵, R⁶ und R⁷ sind - unabhängig voneinander - H, Alkyl oder Alkoxy, jeweils mit 1 bis 9 C-Atomen (linear oder verzweigt),
X⁻ ist ein nicht-nucleophiles Anion, wie Hexafluoroantimonat (SbF₆⁻), -arsenat (AsF₆⁻) und -phosphat (PF₆⁻), Tetraphenylborat (B(C₆H₅)₄⁻), Tetra (perfluorphenyl)-borat (B(C₆F₅)₄⁻) oder Trifluormethansulfonat (CF₃-SO₃⁻).

Die Initiatoren nach der Erfindung sind Derivate des Cumarins (2H-1-Benzopyran-2-on) mit einer Sulfoniumgruppierung in 3-Stellung. Diese Initiatoren absorbieren, gelöst in kationisch polymerisierbaren Monomeren oder Oligomeren, UV-Licht, wobei das Absorptionsmaximum im Bereich von etwa 300 bis 400 nm liegt. Als Folge der UV-Belichtung werden reaktive Kationen bzw. Protonen gebildet. Diese reaktiven Spezies werden auch bei einer Temperaturerhöhung freigesetzt, d.h. dann, wenn die Initiatoren, gelöst in kationisch polymerisierbaren Monomeren oder Oligomeren, einer Temperaturbehandlung ausgesetzt werden.

Die neuen Initiatoren können vorteilhaft in der Weise hergestellt werden, daß in einem ersten Schritt ω-Halogen-3-acetylcumarin, das gegebenenfalls die gewünschten Substituenten (R³ bis R⁷) aufweist, mit einem Dialkylsulfid (mit R¹ und R²), das eine cyclische Verbindung sein kann (z.B.: R¹ + R² = (CH₂)₄), umgesetzt wird; dabei entsteht das entsprechende Dialkylsulfoniumhalogenid. Diese Reaktion kann sowohl in einem geeigneten Lösemittel durchgeführt werden als auch in Substanz. Vorteilhaft wird ein Lösemittel verwendet, in dem die beiden Ausgangsverbindungen homogen löslich sind und aus dem das Sulfoniumsalz ausfällt. Für die Umsetzung können sowohl ω-Chlor- als auch ω-Brom-3-acetylcumarine eingesetzt werden; bevorzugt werden die ω-Bromderivate. Das entstandene Dialkylsulfoniumhalogenid wird üblicherweise isoliert und durch Umkristallisation gereinigt. In einem zweiten Schritt wird dann das Halogenidion gegen ein nicht-nucleophiles Anion ausgetauscht. Dazu wird das Dialkylsulfoniumhalogenid beispielsweise in Methanol gelöst und mit einer Lösung einer äquimolaren Menge von Natriumhexafluoroantimonat in Methanol versetzt. Das Sulfoniumhexafluoroantimonat fällt dabei aus und kann abfiltriert und umkristallisiert werden.

Die zur Synthese der Initiatoren eingesetzten ω-Halogen-3-acetylcumarine können durch eine Kondensationsreaktion aus dem entsprechenden Salicylaldehyd und Acetessigsäureethylester hergestellt werden. Diese Reaktion kann in einer alkoholischen Lösung mit Pyridin als Katalysator bei ca. 60°C durchgeführt werden. Anschließend erfolgt dann die Halogenierung des Acetylrestes in an sich bekannter Weise, beispielsweise mit Brom in einer etherischen Lösung.

Die Initiatoren nach der Erfindung können prinzipiell immer dann eingesetzt werden, wenn durch Kationen bzw. Protonen eine Reaktion bewirkt wird. Dies ist insbesondere bei der kationischen Polymerisation von dazu befähigten Monomeren und Oligomeren der Fall. Derartige Verbindungen sind beispielsweise aus der EP 0 126 712 B1 bekannt.

Kationisch härtbare Reaktionsharzmischungen nach der Erfindung enthalten ein kationisch polymerisierbares Monomer und/ oder Oligomer (Komponente A) und - bezogen auf die Komponente A - 0,01 bis 10 Masse-% des Initiators; die Monomeren bzw. Oligomeren können auch im Gemisch vorliegen. Zur Modifizierung der Verarbeitungs- und Formstoffeigenschaften können die Mischungen Zusatzstoffe, wie mineralische und organische Füllstoffe, Farbstoffe, Pigmente, Stabilisatoren, Thixotropiermittel, Benetzungsmittel und Haftvermittler, enthalten.

Die Komponente A ist vorzugsweise eine oxiranhaltige, d.h. epoxyfunktionelle Verbindung oder eine vinyletherfunktionelle Verbindung. Geeignete oxiranhaltige Verbindungen sind insbesondere epoxidierte Terpene oder α-Alkene, cycloaliphatische Epoxide, Epoxyalkohole, Glycidylether und epoxyfunktionalisierte Silicone; als besonders vorteilhaft haben sich cycloaliphatische Epoxide erwiesen. Bevorzugt werden Verbindungen mit ≥ 2 Epoxidgruppen pro Molekül eingesetzt.

Als vinyletherfunktionelle Verbindungen kommen grundsätzlich alle vinyletherfunktionalisierten Hydroxylverbindungen in Frage. Geeignete Verbindungen sind insbesondere Cyclohexandimethyloldivinylether, Triethylenglykoldivinylether, Butandioldivinylether, Bis(4-vinyloxybutyl)-isophthalat, Bis-(4-vinyloxybutyl)-succinat, Bis(4-vinyloxymethylcyclohexylmethyl)-glutarat und Hydroxybutylmonovinylether oder vinyletherfunktionalisierte Hydroxypolyurethane mit aliphatischer oder aromatischer Grundstruktur. Bevorzugt sind Vinylether mit 2 2 Vinylethergruppen pro Molekül.

Die Reaktionsharzmischungen können zusätzlich noch hydroxylgruppenhaltige Verbindungen enthalten, d.h. polyfunktionelle Hydroxylverbindungen, die im Rahmen einer Kettenübertragungsreaktion am kationischen Reaktionsmechanismus beteiligt sind. Derartige Verbindungen sind insbesondere Polyalkylenpolyole, Polyoxyalkylenpolyole und cycloaliphatische Hydroxylverbindungen; bevorzugt sind polyfunktionelle Hydroxylverbindungen mit ≥ zwei Hydroxylgruppen pro Molekül. Die Verwendung hydroxylgruppenhaltiger Verbindungen erweist sich als vorteilhaft, um die Reaktivität und den erreichbaren Reaktionsumsatz zu erhöhen und die entstehenden Formstoffe zu elastifizieren.

Die Reaktionsharzmischungen nach der Erfindung können sowohl durch Bestrahlung mit UV-Licht als auch thermisch gehärtet werden. In lichtabgeschatteten Bereichen oder solchen Bereichen, die - verfahrensbedingt - durch UV-Belichung nur teilweise ausgehärtet werden, kann die Härtung - gleichzeitig mit der UV-Bestrahlung oder in einem nachfolgenden Prozeß - durch eine Temperaturerhöhung erfolgen. Die Härtungstemperatur liegt im allgemeinen zwischen 80 und 200°C, vorzugsweise etwa bei 80 bis 150°C.

Zur UV-Belichtung können prinzipiell alle üblichen UV-Quellen eingesetzt werden, wie Xenon-, Wolfram-, Quecksilber- und Metallhalogenidstrahler; ferner ist der Einsatz von UV-Lasern möglich. Die Laserstrahlung kann mit Hilfe eines optischen Systems fokussiert sein; die UV-Emission kann sowohl kontinuierlich als auch gepulst erfolgen. Bevorzugt ist der Einsatz von UV-Licht im Wellenlängenbereich von 300 bis 400 nm. Es ist möglich, Schichten aus den Reaktionsharzmischungen vollflächig durch UV-Bestrahlung auszuhärten oder nur lokal begrenzte Bereiche. Die lokale Begrenzung der Härtung kann beispielsweise durch Belichtung über eine Maske erreicht werden. Eine weitere Möglichkeit besteht darin, diese Bereiche mit einem computergesteuerten Laserstrahl zu belichten.

Die Reaktionsharzmischungen (aus kationisch polymerisierbaren Monomeren bzw. Oligomeren und Initiatoren nach der Erfindung) eignen sich zur Beschichtung oder Verklebung von Bauteilen, insbesondere von elektronischen Bauelementen und Baugruppen, und zwar vor allem dann, wenn verfahrensbedingt lichtabgeschattete Bereiche auftreten und/oder die Eindringtiefe des UV-Lichtes zu gering ist für eine vollständige Aushärtung. Dies ist beispielsweise dann der Fall, wenn nicht-transparente Teile verklebt werden; durch UV-Belichtung der lichtzugänglichen Randbereiche kann eine Fixierung und durch einen thermischen Prozeß auch eine Härtung zwischen den Fügepartnern erreicht werden. Reaktionsharzmischungen, die zur Modifizierung der Formstoffeigenschaften lichtstreuende oder -absorbierende Zusätze, wie Füllstoffe, Farbstoffe, Pigmente und Stabilisatoren, enthalten, können durch UV-Bestrahlung oberflächlich oder teilweise gehärtet werden; eine vollständige Härtung ist dann wiederum durch einen thermischen Prozeß möglich.

Die Reaktionsharzmischungen können ferner zur Erzeugung von Strukturen dienen. Dazu wird durch ein geeignetes Verfahren eine Schicht aus der Reaktionsharzmischung hergestellt und diese Schicht durch eine Maske oder mittels eines Laserstrahls belichtet. Die unbelichteten Bereiche werden dann mit einem geeigneten Lösemittel herausgelöst.

Vorzugsweise werden die Reaktionsharzmischungen nach der Erfindung zur stereolithographischen Herstellung dreidimensionaler Gebilde eingesetzt, wobei anhand von 3D-CAD-Daten beliebig komplizierte Kunststoffmodelle realisiert werden können. Dazu wird (in einem Behälter) eine dünne Schicht der Reaktionsharzmischung mittels eines Lasers bildmäßig belichtet und dabei an den Stellen gehärtet, die der unteren Teilfläche des herzustellenden Modells entsprechen. Dabei entsteht eine erste Schicht der dreidimensionalen Struktur. Anschließend wird über dieser ersten Schicht eine weitere dünne Schicht der Reaktionsharzmischung ausgebildet und dann entsprechend belichtet, d.h. gehärtet. Dabei entsteht eine zweite Schicht der dreidimensionalen Struktur, die sich mit der ersten Schicht verbindet. Diese Verfahrensschritte werden so oft wiederholt, bis die dreidimensionale Struktur vollständig schichtweise aufgebaut ist. Zur Erhöhung der Produktivität ist es vorteilhaft, die einzelnen Teilschichten nicht durch eine hohe UV-Dosis vollständig auszuhärten, sondern beispielsweise lediglich die äußere Kontur und im Inneren ein Gitternetz auszuhärten oder die UV-Dosis über die Laserverfahrgeschwindigkeit so einzustellen, daß in der Schicht zwar eine Verfestigung erzielt, aber kein vollständiger Umsatz erreicht wird. In diesen Fällen kann das fertige Modell im Anschluß an den schichtweisen Aufbau und erforderlichenfalls nach einer Reinigung (nach Entnahme aus dem Behälter) durch Temperung und/oder UV-Belichtung auch im Inneren vollständig ausgehärtet werden.

Anhand von Ausführungsbeispielen soll die Erfindung noch näher erläutert werden (Fp = Schmelzpunkt).

### Beispiel 1

Synthese von S-[2-(Benzo[b]pyran-2-on-3-yl)-2-oxo]-ethylthiolanium-hexafluoroantimonat (Initiator 1)
a) Herstellung von ω-Brom-3-acetylcumarin
Zu einer Lösung von 10 g 3-Acetylcumarin (53 mmol) in 250 ml Diethylether wird unter Rühren eine Lösung von 8 g Br₂ in 50 ml Diethylether zugetropft und dann eine weitere Stunde gerührt. Das im Verlauf der Umsetzung ausfallende ω-Brom-3-acetylcumarin wird abgesaugt, mit Diethylether gewaschen und im Vakuum getrocknet.

| | |
|---|---|
| Fp | 163°C |
| Ausbeute | 13 g (92 %) |

b) Herstellung von S-[2-(Benzo[b]pyran-2-on-3-yl)-2-oxo]-ethyl-thiolanium-bromid
5g ω-Brom-3-acetylcumarin (19 mmol) werden mit der äquimolaren Menge Tetrahydrothiophen in 50 ml Aceton versetzt, dann wird bei Raumtemperatur eine Stunde gerührt. Nach etwa 24 h wird der ausgefallene Niederschlag abgesaugt, mit kaltem Aceton gewaschen und im Vakuum getrocknet.
c) Anionenaustausch
Zur Durchführung des Anionenaustausches wird das Sulfoniumbromid in möglichst wenig Methanol gelöst und mit der äquimolaren Menge Natriumhexafluoroantimonat (Na[SbF₆]), das unter leichtem Erwärmen in Methanol gelöst wird, versetzt. Der ausgefallene Niederschlag wird abgesaugt und in Methanol bzw. in einem Methanol/Aceton-Lösemittelgemisch mehrmals umkristallisiert, bis die Halogenidprobe mit AgNO₃ negativ ist.

| | |
|---|---|
| Fp | > 240°C (unter Zersetzung) |
| Ausbeute | 70 % |

### Beispiel 2

### Synthese von S-[2-(8-Methoxy-benzo[b]pyran-2-on-3-yl)-2-oxo]-ethyl-thiolanium-hexafluoroantimonat (Initiator 2)

a) Herstellung von 3-Acetyl-(8-methoxy)-cumarin
7,6 g 3-Methoxysalicylaldehyd (50 mmol) und 7,8 g Acetessigsäureethylester (60 mmol) werden in 50 ml Ethanol gelöst, und nach Zugabe einer katalytischen Menge Pyridin (ca. 10 Tropfen) wird dann 1 h auf 60°C erwärmt. Im Verlauf der Umsetzung fällt das gebildete 3-Acetyl-(8-methoxy)-cumarin nahezu quantitativ aus.

| | |
|---|---|
| Fp | 169°C |
| Ausbeute | 9,8 g (90 %) |

b) Herstellung von ω-Brom-3-acetyl-(8-methoxy)-cumarin
Die Bromierung von 3-Acetyl-(8-methoxy)-cumarin zu ω-Brom-3-acetyl-(8-methoxy)-cumarin wird wie in Beispiel 1a durchgeführt.

| | |
|---|---|
| Fp | 165°C |
| Ausbeute | 81 % |

c) Herstellung von S-[2-(8-Methoxy-benzo[b]pyran-2-on-3-yl)-2-oxo]-ethyl-thiolanium-bromid
Die Umsetzung zum Sulfoniumbromid durch Alkylierung von Tetrahydrothiophen mit ω-Brom-3-acetyl-(8-methoxy)-cumarin erfolgt wie in Beispiel 1b.
d) Anionenaustausch
Der Anionenaustausch erfolgt wie in Beispiel 1c.

| | |
|---|---|
| Fp | 234°C |
| Ausbeute | 40 % |

### Beispiel 3

### Synthese von S-[2-(7-Methoxy-benzo[b]pyran-2-on-3-yl)-2-oxo]-ethyl-thiolanium-hexafluoroantimonat (Initiator 3)

a) Herstellung von 3-Acetyl-(7-methoxy)-cumarin
Es erfolgt eine Umsetzung entsprechend Beispiel 2a unter Verwendung von 4-Methoxy-salicylaldehyd.
b) Herstellung von ω-Brom-3-acetyl-(7-methoxy)-cumarin
Die Bromierung von 3-Acetyl-(7-methoxy)-cumarin zu ω-Brom-3-acetyl-(7-methoxy)-cumarin wird wie in Beispiel 1a durchgeführt.

| | |
|---|---|
| Fp. | 208-210°C |
| Ausbeute | 75% |

c) Herstellung von S-[2-(7-Methoxy-benzo[b]pyran-2-on-3-yl)-2-oxo]-ethyl-thiolanium-hexafluoroantimonat
Zu 3 g ω-Brom-3-acetyl-(7-methoxy)-cumarin (10 mmol) werden 2,7 g Tetrahydrothiophen (30 mmol) gegeben, und dann wird im Wasserbad innerhalb von 30 min auf 50°C erwärmt. Anschließend werden 150 ml Methanol zugegeben, und dann wird weitere 30 min bei 50°C gerührt. Nach dem Abkühlen auf Raumtemperatur wird eine Lösung von 2,6 g Natriumhexafluoroantimonat (10 mmol) in 30 ml Methanol zugegeben; das Sulfoniumhexafluoroantimonat fällt dabei aus. Der ausgefallene Niederschlag wird abgesaugt und in Methanol mehrmals umkristallisiert, bis die Halogenidprobe mit AgNO₃ negativ ist.

| | |
|---|---|
| Fp | 203°C |
| Ausbeute | 3,8 g (70%) |

### Beispiel 4

### Synthese von S-[2-(6-Methoxy-benzo[b]pyran-2-on-3-yl)-2-oxo]-ethyl-thiolanium-hexafluoroantimonat (Initiator 4)

a) Herstellung von 3-Acetyl-(6-methoxy)-cumarin
Es erfolgt eine Umsetzung entsprechend Beispiel 2a unter
Verwendung von 5-Methoxy-salicylaldehyd.

| | |
|---|---|
| Fp | 174°C |
| Ausbeute | 95 % |

b) Herstellung von ω-Brom-3-acetyl-(6-methoxy)-cumarin
Die Bromierung von 3-Acetyl-(6-methoxy)-cumarin zu ω-Brom-3-acetyl-(6-methoxy)-cumarin wird wie in Beispiel 1a durchgeführt.

| | |
|---|---|
| Fp | 142°C |
| Ausbeute | 64 % |

c) Herstellung von S-[2-(6-Methoxy-benzo[b]pyran-2-on-3-yl)-2-oxo]-ethyl-thiolanium-bromid
Die Umsetzung zum Sulfoniumbromid durch Alkylierung von Tetrahydrothiophen mit ω-Brom-3-acetyl-(6-methoxy)-cumarin erfolgt wie in Beispiel 1b.
d) Anionenaustausch
Der Anionenaustausch erfolgt wie in Beispiel 1c.

| | |
|---|---|
| Fp | 243-245°C |
| Ausbeute | 42 % |

Tabelle 1 gibt einen Überblick über die Substituenten und über die Reinheit der Verbindungen nach den Beispielen 1 bis 4, welche folgende Struktur aufweisen:

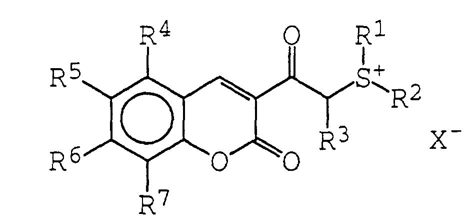

**Tabelle 1**

| | Initiator 1 | Initiator 2 | Initiator 3 | Initiator 4 |
|---|---|---|---|---|
| R¹ | R¹ und R² bilden zusammen eine Tetramethylengruppierung -(CH₂)₄- | | | |
| R² | | | | |
| R³ | -H | -H | -H | -H |
| R⁴ | -H | -H | -H | -OCH₃ |
| R⁵ | -H | -H | -OCH₃ | -H |
| R⁶ | -H | -OCH₃ | -H | -H |
| R⁷ | -H | -H | -H | -H |
| X⁻ | SbF₆⁻ | SbF₆⁻ | SbF₆⁻ | SbF₆⁻ |

| Elementaranalyse | | | | |
|---|---|---|---|---|
| C [%] (ber.) | 35,2 | 35,5 | 35,5 | 35,5 |
| C [%] (gef.) | 34,9 | 34,7 | 35,2 | 34,0 |
| H [%] (ber.) | 2,94 | 3,14 | 3,14 | 3,14 |
| H [%] (gef.) | 2,96 | 3,2 | 3,5 | 3,3 |
| S [%] (ber.) | 6,3 | 5,9 | 5,9 | 5,9 |
| S [%] (gef.) | 6,3 | 6,1 | 6,1 | 6,0 |
| "ber." = berechnet, "gef." = gefunden | | | | |

Tabelle 2, in der die UV-Absorption in Dimethylformamid wiedergegeben ist, zeigt anhand der Extinktionskoeffizienten, daß die Verbindungen nach den Beispielen 1 bis 4 in der Lage sind, bei 366 nm (Quecksilberlinie) effektiv UV-Licht zu absorbieren. Ein handelsübliches Triarylsulfoniumsalz (UVI 6974, Union Carbide) absorbiert dagegen bei dieser Wellenlänge nur noch in vernachlässigbarem Maße (Vergleichsbeispiel).

**Tabelle 2**

| | Extinktionskoeffizient bei 366 nm [l/g·cm] | Absorptionsmaximum | |
|---|---|---|---|
| | | Wellenlänge [nm] | Extinktionskoeffizient [l/g·cm] |
| Triarylsulfoniumsalz (Vergleichsbeispiel) | 0,8 | 308 | 9,9 |
| Initiator 1 | 13,8 | 313 / 349 | 23,5 / 16,9 |
| Initiator 2 | 9,4 | 331 | 27,2 |
| Initiator 3 | 36,5 | 375 | 39 |
| Initiator 4 | 8,2 | 311 / 391 | 23,1 / 10,6 |

Die nachfolgenden Beispiele 5 bis 11 zeigen, daß die Initiatoren nach der Erfindung vorteilhaft zur Härtung kationisch polymerisierbarer Reaktionsharzmischungen eingesetzt werden können. Die Zusammensetzung der Harzmischungen (in Masseteilen) ist in Tabelle 3 angegeben.

### Beispiele 5 bis 8

Zur Herstellung einer Harzbasis werden gleiche Masseteile Bisphenol-A-diglycidylether und Cyclohexan-dimethyloldivinylether unter Erwärmen auf ca. 50°C unter Rühren gelöst.

Aus dem jeweiligen Initiator und 1,2-Propylencarbonat wird eine Lösung hergestellt, zu der die der Zusammensetzung nach Tabelle 3 entsprechende Menge an Harzbasis gegeben wird. Die erhaltene Reaktionsharzmischung wird dann bei Raumtemperatur unter Lichtausschluß gerührt und homogenisiert.

Die in Tabelle 4 wiedergegebenen Ergebnisse bezüglich der thermischen Reaktivität der Reaktionsharzmischungen zeigen, daß die Mischungen aus den Initiatoren nach der Erfindung und kationisch polymerisierbaren Komponenten lagerstabil sind und rein thermisch gehärtet werden können.

### Beispiele 9 bis 11

Zur Herstellung einer Harzbasis werden 95 Masseteile Bis-(epoxycyclohexyl-methyl)-adipat und 5 Masseteile Trimethylolpropan unter Erwärmen auf ca. 50°C unter Rühren gelöst. Aus dem jeweiligen Initiator und 1,2-Propylencarbonat wird eine Lösung hergestellt, zu der die der Zusammensetzung nach Tabelle 3 entsprechende Menge an Harzbasis gegeben wird. Die erhaltene Reaktionsharzmischung wird dann bei Raumtemperatur unter Lichtausschluß gerührt und homogenisiert.

Die in Tabelle 5 wiedergegebenen Ergebnisse bezüglich der Reaktivität der Reaktionsharzmischungen bei UV-Bestrahlung zeigen, daß die Mischungen aus den Initiatoren nach der Erfindung und kationisch polymerisierbaren Komponenten durch UV-Bestrahlung gehärtet werden können.

**Tabelle 3**

| Beispiel | 5 | 6 | 7 | 8 | 9 | 10 | 11 |
|---|---|---|---|---|---|---|---|
| Bisphenol-A-diglycidylether | 50 | 50 | 50 | 50 | - | - | - |
| Cyclohexan-dimethylol-divinylether | 50 | 50 | 50 | 50 | - | - | - |
| Bis(epoxycyclohexyl-methyl)-adipat | - | - | - | - | 95 | 95 | 95 |
| Trimethylolpropan | - | - | - | - | 5 | 5 | 5 |
| 1,2-Propylencarbonat | 1,6 | 1,6 | 1,6 | 1,6 | 1,6 | 1,6 | 1,6 |
| Initiator 1 | 0,4 | - | - | - | - | - | - |
| Initiator 2 | - | 0,4 | - | - | 0,4 | - | - |
| Initiator 3 | - | - | 0,4 | - | - | 0,4 | - |
| Initiator 4 | - | - | - | 0,4 | - | | 0,4 |

**Tabelle 4**

| Beispiel | 5 | 6 | 7 | 8 |
|---|---|---|---|---|
| Lagerstabilität (Zeit bis zur Viskositätsverdoppelung) | > 6 Monate | > 6 Monate | > 6 Monate | > 6 Monate |

| Ergebnisse aus DSC-Untersuchungen (Heizrate 10 K/min) | | | | |
|---|---|---|---|---|
| Onset [°C] | 111 | 112 | 108 | 110 |
| Peakmaximum [°C] | 118 und 188 | 124 und 188 | 118 und 186 | 120 und 183 |
| Enthalpie [J/g] | 430 | 420 | 410 | 470 |

**Tabelle 5**

| Beispiel | 9 | 10 | 11 |
|---|---|---|---|
| Ergebnisse aus Photo-DSC-Untersuchungen (isothermer Meßlauf bei 40°C; Belichtung während der Messung mit UV-Licht der Wellenlänge 351 nm, d.h. Linie des ArgonIonen-Lasers) | | | |
| Onset [s] | 1,62 | 1,38 | 2,1 |
| Zeit bis zum Peakmaximum [s] | 7,92 | 9,0 | 11,6 |
| Peakhöhe [W/g] | 3,63 | 3,48 | 1,99 |
| Enthalpie [J/g] | 161 | 162 | 149 |

## Patentansprüche

1. Initiatoren für die kationische Polymerisation, **gekennzeichnet durch** folgende Struktur: 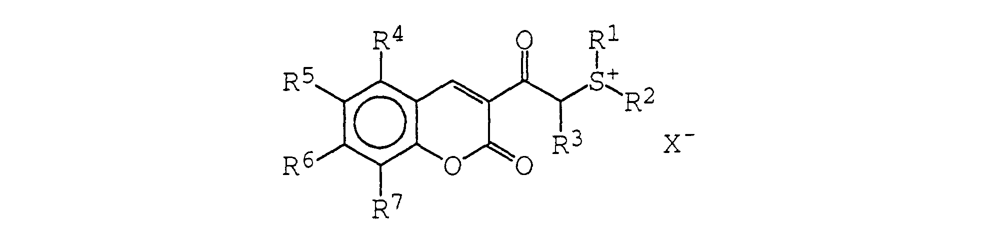 wobei folgendes gilt:
R¹ und R² sind Alkyl mit 1 bis 9 C-Atomen oder Cycloalkyl mit 4 bis 9 C-Atomen oder bilden zusammen eine divalente aliphatische Gruppierung mit 4 bis 7 C-Atomen,
R³ ist H oder Alkyl mit 1 bis 9 C-Atomen,
R⁴, R⁵, R⁶ und R⁷ sind H, Alkyl mit 1 bis 9 C-Atomen oder Alkoxy mit 1 bis 9 C-Atomen,
X⁻ ist ein nicht-nucleophiles Anion, wie Hexafluoroantimonat, -arsenat und -phosphat, Tetraphenylborat, Tetra(perfluorphenyl)-borat oder Trifluormethansulfonat.

2. Reaktionsharzmischungen, enthaltend
- ein kationisch polymerisierbares Monomer und/oder Oligomer (Komponente A) und
- 0,01 bis 10 Masse-%, bezogen auf die Komponente A, eines Initiators nach Anspruch 1 (Komponente B) sowie
- gegebenenfalls Füllstoff, Pigment und/oder Additiv.

3. Reaktionsharzmischungen nach Anspruch 2, **dadurch gekennzeichnet, daß** die Komponente A eine oxiranhaltige Verbindung ist, insbesondere eine Verbindung mit mindestens zwei Epoxidgruppen pro Molekül.

4. Reaktionsharzmischungen nach Anspruch 3, **dadurch gekennzeichnet, daß** die oxiranhaltige Verbindung ein epoxidiertes Terpen oder α-Alken, ein cycloaliphatisches Epoxid, ein Epoxyalkohol, ein Glycidylether oder ein epoxyfunktionalisiertes Silicon ist.

5. Reaktionsharzmischungen nach Anspruch 2, **dadurch gekennzeichnet, daß** die Komponente A eine vinyletherfunktionelle Verbindung ist.

6. Reaktionsharzmischungen nach Anspruch 5, **dadurch gekennzeichnet, daß** die vinyletherfunktionelle Verbindung mindestens zwei Vinylethergruppen pro Molekül aufweist.

7. Reaktionsharzmischungen nach einem der Ansprüche 2 bis 6, **dadurch gekennzeichnet, daß** sie zusätzlich eine polyfunktionelle Hydroxylverbindung enthalten.

8. Verwendung der Reaktionsharzmischungen nach einem oder mehreren der Ansprüche 2 bis 7 zur Beschichtung oder Verklebung von nicht-transparenten Bauteilen, insbesondere von elektronischen Bauelementen und Baugruppen.

9. Verwendung der Reaktionsharzmischungen nach einem oder mehreren der Ansprüche 2 bis 7 zur Erzeugung von Strukturen.

10. Verwendung der Reaktionsharzmischungen nach einem oder mehreren der Ansprüche 2 bis 7 zur stereolithographischen Herstellung dreidimensionaler Gebilde.

## Claims

1. Initiators for cationic polymerization, **characterized by** the following structure: 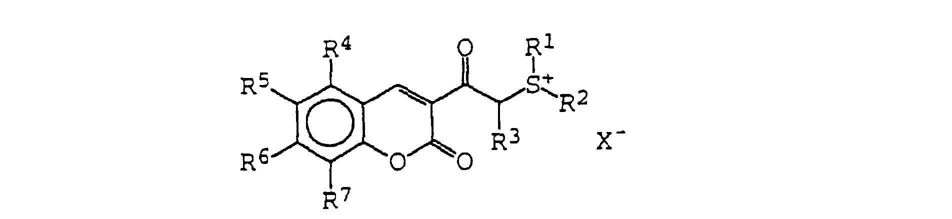 where:
R¹ and R² are alkyl having from 1 to 9 carbon atoms or cycloalkyl having from 4 to 9 carbon atoms, or together form a bivalent aliphatic group having from 4 to 7 carbon atoms,
R³ is H or alkyl having from 1 to 9 carbon atoms,
R⁴, R⁵, R⁶ and R⁷ are H, alkyl having from 1 to 9 carbon atoms or alkoxy having from 1 to 9 carbon atoms,
X⁻ is a non-nucleophilic anion, such as hexafluoroantimonate, -arsenate or -phosphate, tetraphenylborate, tetra(perfluorophenyl)borate or trifluoromethanesulphonate.

2. Reactive resin mixtures, comprising
- a cationically polymerizable monomer and/or oligomer (component A) and
- from 0.01 to 10% by weight based on component A, of an initiator according to Claim 1 (component B) and also
- where appropriate, filler, pigment and/or additive.

3. Reactive resin mixtures according to Claim 2, **characterized in that** component A is an oxirane-containing compound, in particular a compound having at least two epoxy groups per molecule.

4. Reactive resin mixtures according to Claim 3, **characterized in that** the oxirane-containing compound is an epoxidized terpene or α-alkene, a cycloaliphatic epoxide, an epoxy alcohol, a glycidyl ether or an epoxy-functionalized silicone.

5. Reactive resin mixtures according to Claim 2, **characterized in that** component A is a vinylether-functional compound.

6. Reactive resin mixtures according to Claim 5, **characterized in that** the vinyl-ether-functional compound has at least two vinyl ether groups per molecule.

7. Reactive resin mixtures according to any of Claims 2 to 6, **characterized in that** they also comprise a polyfunctional hydroxyl compound.

8. Use of the reactive resin mixtures according to one or more of Claims 2 to 7 for the coating or adhesive bonding of non-transparent components, in particular of electronic components or modules.

9. Use of the reactive resin mixtures according to one or more of Claims 2 to 7 for producing structures.

10. Use of the reactive resin mixtures according to one or more of Claims 2 to 7 for the stereolithographic production of three-dimensional constructions.

## Revendications

1. Initiateurs pour la polymérisation cationique, **caractérisés par** la structure suivante 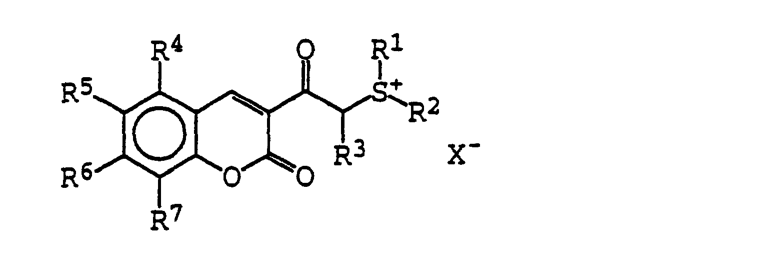 dans laquelle :
R¹ et R² sont, indépendamment l'un de l'autre, un alkyle comptant de 1 à 9 atomes de carbone ou un cycloalkyle comptant de 4 à 9 atomes de carbone ou forment ensemble un groupe aliphatique divalent comptant de 4 à 7 atomes de carbone,
R³ représente H ou un groupe alkyle comptant de 1 à 9 atomes de carbone,
R⁴, R⁵, R⁶ et R⁷ représentent, indépendamment l'un de l'autre, H, un alkyle ou un alkoxy comptant chaque fois de 1 à 9 atomes de carbone,
X⁻ est un anion non nucléophile, par exemple l'hexafluoroantimoniate, un hexafluoroarséniate et un hexafluorophosphate, le tétraphénylborate, le tétra(perfluorophényl)-borate ou le trifluorométhanesulfonate.

2. Mélanges de résines réactives, contenant :
- un monomère et/ou oligomère polymérisable cationiquement (composant A), et
- de 0,01 à 10 % en masse, calculés par rapport au composant A, d'un initiateur selon la revendication 1 (composant B) ainsi que
- éventuellement des charges, des pigments et/ou des additifs.

3. Mélanges de résines réactives selon la revendication 2, **caractérisés en ce que** le composant A est un composé contenant un oxyrane, et en particulier un composé présentant au moins deux groupes époxy par molécule.

4. Mélanges de résines réactives selon la revendication 3, **caractérisés en ce que** le composé contenant un oxyrane est un terpène ou α-alcène époxydé, un époxy cycloaliphatique, un alcool époxylé, un éther de glycidyle ou un silicone à fonctionnalités époxy.

5. Mélanges de résines réactives selon la revendication 2, **caractérisés en ce que** le composant A est un composé à fonctionnalités éther de vinyle.

6. Mélanges de résines réactives selon la revendication 5, **caractérisés en ce que** le composé à fonctionnalités éther de vinyle présente au moins deux groupes éther de vinyle par molécule.

7. Mélanges de réactions selon l'une des revendication 2 à 6, **caractérisés en ce qu'**ils contiennent en supplément un composé hydroxylé polyfonctionnel.

8. Utilisation des mélanges de résines réactives selon l'une ou plusieurs des revendications 2 à 7 pour le revêtement ou le collage de composants non transparents, en particulier de composants et du groupe de composants électroniques.

9. Utilisation de mélanges de résines réactives selon l'une ou plusieurs des revendications 2 à 7 pour la création de structures.

10. Utilisation de mélanges de résines réactives selon l'une ou plusieurs des revendications 2 à 7 pour la préparation stéréolithographique d'objets tridimensionnels.
